# FASCICULE DE BREVET EUROPEEN

(11) **EP 1 144 357 B1**
(45) Date de publication et mention de la délivrance du brevet: **20.08.2003**
(21) Numéro de dépôt: 00900625.5
(22) Date de dépôt: 20.01.2000
(51) Int. Cl.: C07C 213/08, C07C 219/08

(54) **PROCEDE DE FABRICATION DE SOLUTIONS AQUEUSES DE SELS INSATURES D'AMMONIUM QUATERNAIRE**
VERFAHREN ZUR HERSTELLUNG VON WÄSSRIGEN LÖSUNGEN VON UNGESÄTTIGTEN QUATERNÄREN AMMONIUMSALZEN
METHOD FOR MAKING AQUEOUS SOLUTIONS OF UNSATURATED QUATERNARY AMMONIUM SALTS

(30) Priorité: 21.01.1999 FR 9900642
(43) Date de publication de la demande: 17.10.2001
(73) Titulaire: Atofina, 92800 Puteaux (FR)
(72) Inventeur: RIONDEL, Alain, F-57600 Forbach (FR); HERBST, Gilles, F-57350 Spicheren (FR); ESCH, Marc, F-57800 Freyming-Merlebach (FR)
(74) Mandataire: Rieux, Michel
(86) Numéro de dépôt international: FR0000123
(87) Numéro de publication internationale: WO00043347

(56) Documents cités:
- EP-A- 0 250 325
- EP-A- 0 329 512
- EP-A- 0 818 437
- EP-A- 0 819 671
- WO-A-89/07588

## Description

La présente invention porte sur la fabrication de solutions aqueuses de sels insaturés d'ammonium quaternaire (ci-après dénommés sels quaternaires), répondant à la formule (I) suivante : 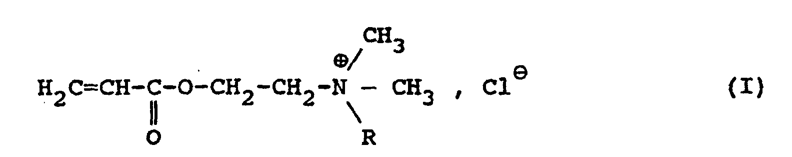 dans laquelle R représente méthyle ou benzyle,
par réaction, en présence d'eau, de l'acrylate de N,N-diméthylaminoéthyle (ADAME) avec un agent quaternisant de formule (II) :

R - Cl (II)

dans laquelle R est tel que défini ci-dessus.

On utilise des solutions aqueuses de sels quaternaires (I) pour préparer des polymères destinés à servir de floculants cationiques dans le traitement des eaux.

Le brevet européen EP-B-250 325 décrit un procédé de préparation de solutions aqueuse de sels quaternaires dont ceux de formule (I), procédé selon lequel, en présence d'au moins un inhibiteur de polymérisation :
- dans une première étape (a), on fait réagir l'ADAME avec 5 à 20% en poids de la quantité pondérale nécessaire à la réaction de l'agent quaternisant, ou, suivant une variante (a'), avec 5 à 20% en poids, par rapport au poids de l'ADAME, d'une solution aqueuse de sels quaternaires, laquelle comprend de 50 à 85% en poids de sels quaternaires ; et
- dans une deuxième étape (b), on ajoute en continu l'eau et l'agent quaternisant jusqu'à l'obtention de la concentration souhaitée de sels quaternaires dans l'eau.

Pendant les étapes (a) et (b), on maintient la température à une valeur comprise entre 30 et 60°C. De plus, pendant les étapes (a) et (b) et en particulier à l'approche de la fin de la réaction, on maintient dans le milieu réactionnel un courant de gaz oxygéné tel que le rapport en volume (ou débit volumétrique) de gaz total à la sortie du réacteur sur le volume (ou débit volumétrique) d'oxygène introduit à l'entrée de ce même réacteur est inférieur à 100.

Ce procédé permet de préparer des solutions aqueuses de sels quaternaires qui ont une stabilité à température ambiante supérieure à un an. Toutefois, on constate, dans ces solutions, une teneur particulièrement élevée d'impuretés, en particulier de 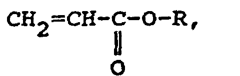 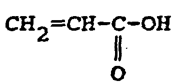 et d'ADAME. En outre, ce procédé nécessite des temps de réaction relativement longs, ce qui représente un inconvénient économique évident.

Dans la demande internationale WO 89/07 588, a alors été proposé un procédé destiné à réduire la formation des impuretés lors de la réaction de quaternisation. Conformément à ce procédé, la réaction est effectuée à une température comprise entre 10 et 80°C, et
(a) dans une première étape, on introduit dans le réacteur la totalité ou une partie de l'agent quaternisant nécessaire à la réaction, cet agent étant à l'état liquide dans les conditions de la réaction,
(b) ensuite, on ajoute l'ADAME, et
(c) dès que 0 à 30% de la stoechiométrie de l'ADAME ont été introduits dans le réacteur, on ajoute en continu et simultanément le reste d'agent quaternisant, le reste d'ADAME et l'eau jusqu'à l'obtention de la concentration souhaitée de sels quaternaires,
(d) et, dans le cas où l'agent quaternisant est introduit à l'état gazeux à la température de réaction, la réaction est effectuée en présence d'oxygène et on impose une pression de manière que l'agent quaternisant soit liquide à la température de réaction, et, en fin de réaction, on diminue progressivement la pression jusqu'à la pression atmosphérique et simultanément on impose un rapport en débit volumétrique de gaz total à la sortie du réacteur sur le débit volumétrique d'oxygène introduit dans le réacteur inférieur à 100.

Le procédé ci-dessus selon WO 89/07 588 apporte des améliorations notables au procédé selon EP-B-250 325. Cependant, il est apparu que la pureté avec laquelle on obtient les sels quaternaires est encore insuffisante. Ainsi, au cours de la réaction de l'ADAME avec CH₃Cl en milieu aqueux, conduisant au sel désigné également dans ce qui suit par l'abréviation ADAMQUAT MC, il se forme, comme impuretés, outre l'acide acrylique (AA) formé par hydrolyse de l'ADAME, le dimère de l'ADAMQUAT MC, représenté par la formule (1) : 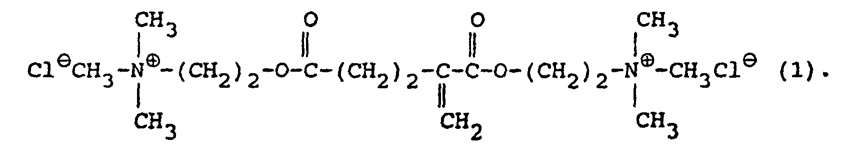 Grâce à une série de tests de réactivité en polymérisation, il a pu être démontré que ces impuretés affectaient la qualité des polymères cationiques dérivés de l'ADAMQUAT.

La Société déposante a donc recherché des conditions opératoires de préparation de solutions aqueuses du sel de formule (I), qui soient capables de minimiser les impuretés précitées, de façon à proposer un sel (I) en solution aqueuse de très haute qualité analytique.

Ce nouveau procédé, qui fait donc l'objet de la présente invention, est caractérisé par le fait que :
(a) dans un réacteur fermé qui contient 5 - 60% de la quantité pondérale de l'ADAME nécessaire à la réaction et qui a été pressurisé par de l'air ou de l'air appauvri à 0,5 à 3 bars, on conduit la réaction en introduisant en continu, à la température de 35 à 65°C, en particulier de 40 à 60°C, d'une part, l'agent quaternisant (II) et, d'autre part, l'eau, et enfin, l'ADAME restant, jusqu'à l'obtention de la concentration souhaitée en sel (I) dans l'eau,
   - le démarrage de l'introduction de l'eau commençant lorsque l'on a ajouté 0 - 30%, en particulier 10 - 20%, de la quantité pondérale nécessaire à la réaction de l'agent quaternisant (II) ;
   - le démarrage de l'introduction de l'ADAME restant commençant lorsque l'on a ajouté 20 - 80%, en particulier 30 - 70%, de la quantité pondérale nécessaire à la réaction de l'agent quaternisant (II) ; et
   - la pression en fin de réaction pouvant atteindre 9 bars, en particulier 4 à 7 bars ; puis
(b) on dépressurise le réacteur tout en maintenant la teneur constante en oxygène par introduction simultanée d'air, et après retour à la pression atmosphérique, on élimine l'agent quaternisant résiduaire, par exemple par strippage à l'air.

Conformément à d'autres caractéristiques particulières du procédé selon l'invention :
- on introduit l'agent quaternisant pendant un laps de temps de 1 - 7 heures, l'eau pendant un laps de temps de 1 - 8 heures, et l'ADAME restant pendant un laps de temps de 2 - 8 heures ;
- on conduit la réaction avec un rapport molaire de l'agent quaternisant à l'ADAME de 1 à 1,1, de préférence de 1 à 1,05 ;
- on conduit la réaction avec un rapport moyen de débit eau / agent quaternisant de 0,2 - 1,5, en particulier de 0,4 - 1, un rapport moyen de débit ADAME restant /agent quaternisant de 2,5 - 5, en particulier de 3 - 4, et un rapport moyen de débit eau / ADAME restant de 0,2 - 1,2, en particulier de 0,3 - 0,9.

Le procédé selon l'invention permet notamment de préparer des solutions aqueuses ayant une concentration en sels quaternaires (I) de 50 à 85% en poids, et contenant des quantités très faibles d'impuretés, comme illustré dans le Tableau 1 ci-après.

Par ailleurs, le procédé selon la présente invention peut être conduit en présence d'au moins un stabilisant, lequel peut être choisi parmi le 3,5-ditert.-butyl-4-hydroxytoluène, l'éther méthylique d'hydroquinone, l'hydroquinone, le catéchol, le tert.-butyl catéchol, la phénothiazine, et les mélanges de ces stabilisants, la teneur en agent(s) stabilisant(s) étant notamment de 20 à 2000 ppm, de préférence de 100 à 1200 ppm, par rapport à la solution aqueuse de sel quaternaire (I).

On peut ajouter en outre au milieu réactionnel au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

D'une manière générale, les agents séquestrants sont ajoutés sous la forme d'une solution aqueuse, car ils sont généralement disponibles sous cette forme. Ainsi, le sel pentasodique de l'acide diéthylène triamine penta acétique commercialisé sous la dénomination VERSENEX 80 se présente sous la forme d'une solution aqueuse à 40% en poids environ.

Les Exemples suivants illustrent la présente invention sans toutefois en limiter la portée. Des ces exemples, les pourcentages sont en poids sauf indication contraire.

### EXEMPLE 1 :

Dans un réacteur en verre de 1 l, à double enveloppe, spécialement conçu pour tenir la pression, équipé d'une sonde de température, d'un agitateur spécifique gaz liquide (turbine avec axe creux), d'une soupape tarée à 10 bars, d'un disque d'explosion et de cannes plongeantes pour l'introduction des différents réactifs, on a chargé 200 g d'ADAME (soit 46,6% de la totalité de l'ADAME). Le réacteur a été fermé, puis pressurisé avec 1 bar d'air appauvri. L'agitation et le chauffage ont été mis en service.

Dès que la température a atteint 40°C (température de procédé = 47°C), on a commencé à introduire CH₃Cl à un débit de 70 g/h. Lorsque 35 g de CH₃Cl ont été introduits, on a démarré l'introduction d'eau à un débit de 28,6 g/h. Après 1 h de réaction, le débit de CH₃Cl a été ramené à 20,9 g/h. L'introduction du reste de l'ADAME (soit 229 g) a débuté après 1,5 h de réaction à un débit de 76,3 g/h. En fin de réaction, le réacteur a été ramené à la pression atmosphérique en utilisant le protocole suivant :
- dégazage du CH₃Cl en excès pendant 30 minutes avec introduction simultanée d'air dans la charge (débit : 3 Nl/h) ;
- retour progressif à la pression atmosphérique,
et les traces de CH₃Cl, éliminées par stripping à l'air (débit : 5 Nl/h) pendant 30 minutes.

Le réacteur a ensuite été mis en refroidissement, puis vidangé. On a récupéré 710 g d'ADAMQUAT MC 80, lequel a été analysé en chromatographie liquide haute performance (HPLC) pour déterminer les teneurs en AA et en composé (1). Les résultats sont rapportés dans le Tableau 1.

Les durées des différentes phases de la réaction étaient les suivantes :
- introduction CH₃Cl : 5,25 h
- introduction H₂O : 5 h
- introduction ADAME : 3 h
- dégazage : 0,5 h
- stripping : 0,5 h
soit une durée totale d'environ 6,75 h.

Les rapports de débit utilisés étaient :
- H₂O/CH₃Cl : 0,41 pendant la première heure, puis
   1,37 pendant le reste de la réaction ;
- H₂O/ADAME : 0,37
- ADAME/CH₃Cl : 3,64.

### EXEMPLE 2 :

On a procédé comme à l'Exemple 1 excepté que l'on a augmenté le débit de CH₃Cl.

Les résultats sont également rapportés dans le Tableau 1.

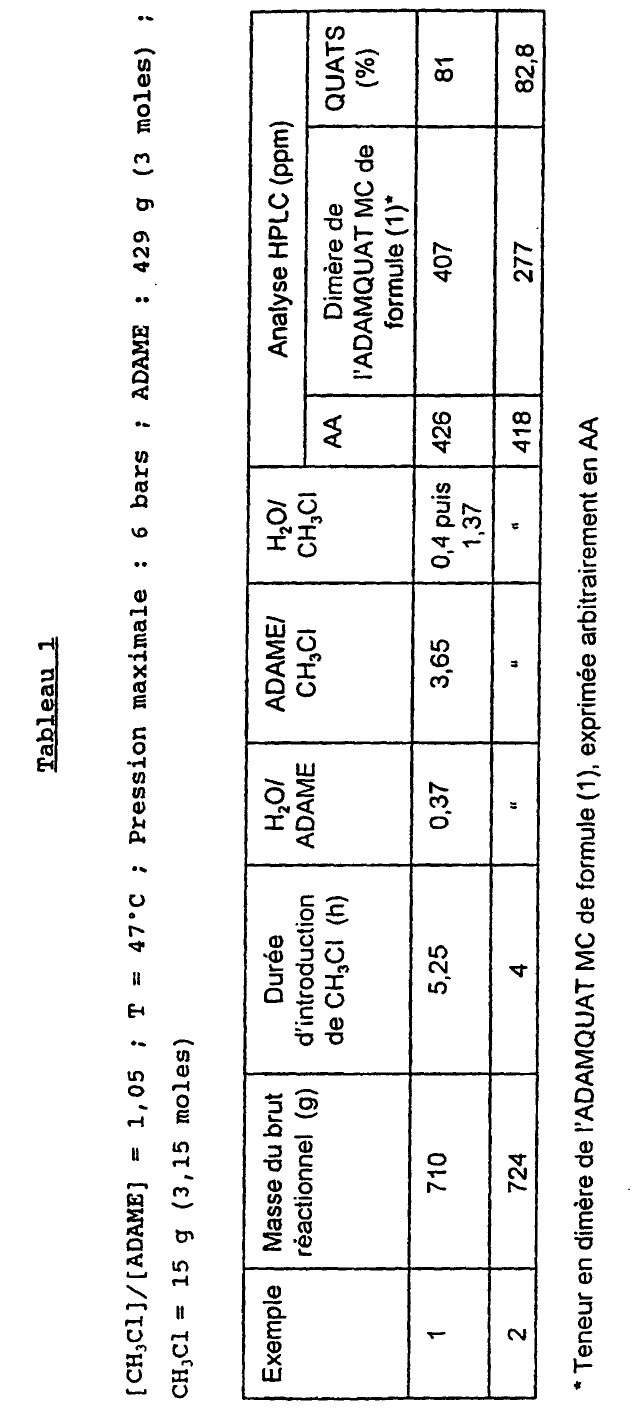

## Revendications

1. Procédé de fabrication de solutions aqueuses de sels insaturés d'ammonium quaternaire répondant à la formule (I) suivante : 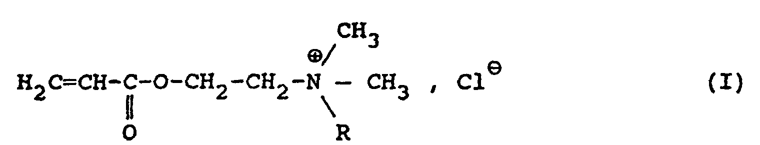 dans laquelle R représente un radical méthyle ou benzyle, par réaction, en présence d'eau, de l'acrylate de N,N-diméthylaminoéthyle (ADAME) avec un agent quaternisant de formule (II) :
R - Cl (II)
dans laquelle R est tel que défini ci-dessus,
**caractérisé par le fait que** :
(a) dans un réacteur fermé qui contient 5 - 60% de la quantité pondérale de l'ADAME nécessaire à la réaction et qui a été pressurisé par de l'air ou de l'air appauvri à 0,5 à 3 bars, on conduit la réaction en introduisant en continu, à la température de 35 à 65°C, d'une part, l'agent quaternisant (II) et, d'autre part, l'eau, et enfin, l'ADAME restant, jusqu'à l'obtention de la concentration souhaitée en sel (I) dans l'eau,
• le démarrage de l'introduction de l'eau commençant lorsque l'on a ajouté 0 - 30% de la quantité pondérale nécessaire à la réaction de l'agent quaternisant (II) ;
• le démarrage de l'introduction de l'ADAME restant commençant lorsque l'on a ajouté 20 - 80% de la quantité pondérale nécessaire à la réaction de l'agent quaternisant (II) ; et
• la pression en fin de réaction pouvant atteindre 9 bars ; puis
(b) on dépressurise le réacteur tout en maintenant la teneur constante en oxygène par introduction simultanée d'air et, après retour à la pression atmosphérique, on élimine l'agent quaternisant résiduaire.

2. Procédé selon la revendication 1, **caractérisé par le fait qu'**on conduit la réaction à une température de 40 à 60°C.

3. Procédé selon l'une des revendications 1 et 2, **caractérisé par le fait qu'**on conduit la réaction avec une pression qui, en fin de réaction, atteint 4 à 7 bars.

4. Procédé selon l'une des revendications 1 à 3, **caractérisé par le fait qu'**on démarre l'introduction de l'eau lorsque l'on a ajouté 10 - 20% de la quantité pondérale nécessaire à la réaction de l'agent quaternisant (II).

5. Procédé selon l'une des revendications 1 à 4, **caractérisé par le fait qu'**on démarre l'introduction de l'ADAME restant lorsque l'on a ajouté 30 - 70% de la quantité pondérale nécessaire à la réaction de l'agent quaternisant (II).

6. Procédé selon l'une des revendications 1 à 5, **caractérisé par le fait que** l'on introduit l'agent quaternisant pendant un laps de temps de 1 - 7 heures, l'eau pendant un laps de temps de 1 - 8 heures, et l'ADAME restant pendant un laps de temps de 2 - 8 heures.

7. Procédé selon l'une des revendications 1 à 6, **caractérisé par le fait que** l'on conduit la réaction avec un rapport molaire de l'agent quaternisant à l'ADAME de 1 à 1,1, de préférence de 1 à 1,05.

8. Procédé selon l'une des revendications 1 à 7, **caractérisé par le fait que** l'on conduit la réaction avec un rapport moyen de débit eau / agent quaternisant de 0,2 - 1,5 ; un rapport moyen de débit ADAME restant / agent quaternisant de 2,5 - 5 ; et un rapport moyen de débit eau /ADAME restant de 0,2 - 1,2.

9. Procédé selon l'une des revendications 1 à 8, **caractérisé par le fait qu'**il conduit à une solution aqueuse ayant une concentration en sel quaternaire (I) de 50 à 85% en poids.

10. Procédé selon l'une des revendications 1 à 9, **caractérisé par le fait qu'**il est conduit en présence d'au moins un stabilisant, choisi notamment parmi le 3,5-ditert.-butyl-4-hydroxytoluène, l'éther méthylique d'hydroquinone, l'hydroquinone, le catéchol, le tert.-butyl catéchol, la phénothiazine, et les mélanges de ces stabilisants, la teneur en agent(s) stabilisant(s) étant notamment de 20 à 2000 ppm, de préférence de 100 à 1200 ppm par rapport à la solution aqueuse de sel quaternaire (I).

11. Procédé selon la revendication 10, **caractérisé par le fait qu'**il est conduit en outre en présence d'au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

12. Procédé selon l'une des revendications 1 à 11, **caractérisé par le fait que** l'on élimine l'agent quaternisant résiduaire par strippage à l'air.

## Patentansprüche

1. Verfahren zur Herstellung von wässrigen Lösungen von ungesättigten quartären Ammoniumsalzen der folgenden Formel (I): 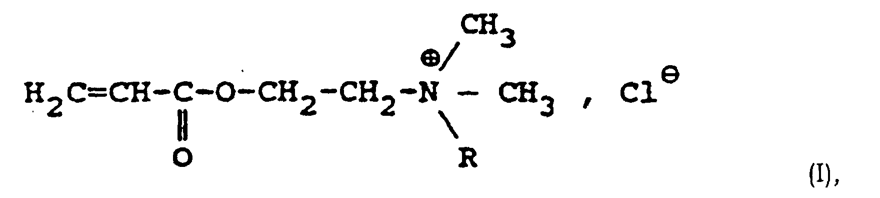 worin R Methyl oder Benzyl bedeutet, durch Umsetzung von N,N-Dimethylaminoethylacrylat (ADAME) mit einem Quaternisierungsmittel der folgenden Formel (II) in Gegenwart von Wasser:
R - Cl (II),
worin R die oben angegebenen Bedeutungen aufweist, **dadurch gekennzeichnet, dass**:
(a) in einem geschlossenen Reaktor, der 5 bis 60% der für die Umsetzung erforderlichen Gewichtsmenge ADAME enthält und der mit Luft oder abgereicherter Luft unter einen Druck von 0,5 bis 3 bar gesetzt ist, die Reaktion durchgeführt wird, indem kontinuierlich bei einer Temperatur von 35 bis 65° C einerseits das Quaternisierungsmittel (II) und andererseits Wasser und schließlich das restliche ADAME zugegeben wird, bis die gewünschte Konzentration des Salzes (I) in Wasser erreicht ist, wobei
• mit der Zugabe des Wassers begonnen wird, wenn O bis 30% der für die Umsetzung erforderlichen Gewichtsmenge des Quaternisierungsmittels (II) zugegeben sind;
• mit der Zugabe des restlichen ADAME begonnen wird, wenn 20 bis 80% der für die Umsetzung erforderlichen Gewichtsmenge des Quaternisierungsmittels (II) zugegeben sind; und
• der Druck am Ende der Reaktion 9 bar erreichen kann; und anschließend
(b) der Druck in dem Reaktor gesenkt und gleichzeitig der Sauerstoffgehalt konstant gehalten wird, indem gleichzeitig Luft zugeführt wird, und nach Rückkehr auf Atmosphärendruck das restliche Quaternisierungsmittel entfernt wird.

2. Verfahren nach Anspruch 1, **dadurch gekennzeichnet, dass** die Reaktion bei einer Temperatur von 40 bis 60° C durchgeführt wird.

3. Verfahren nach einem der Ansprüche 1 und 2, **dadurch gekennzeichnet, dass** die Umsetzung bei einem Druck durchgeführt wird, der am Ende der Umsetzung 4 bis 7 bar erreicht.

4. Verfahren nach einem der Ansprüche 1 bis 3, **dadurch gekennzeichnet, dass** mit der Zugabe von Wasser begonnen wird, wenn 10 bis 20% der für die Umsetzung erforderlichen Menge des Quaternisierungsmittel (II) zugegeben sind.

5. Verfahren nach einem der Ansprüche 1 bis 4, **dadurch gekennzeichnet, dass** mit der Zugabe des restlichen ADAME begonnen wird, wenn 30 bis 70% der für die Umsetzung erforderlichen Gewichtsmenge des Quaternisierungsmittels (II) zugegeben sind.

6. Verfahren nach einem der Ansprüche 1 bis 5, **dadurch gekennzeichnet, dass** das Quaternisierungsmittel während einer Zeitspanne von 1 bis 7 Stunden, das Wasser während einer Zeitspanne von 1 bis 8 Stunden und das restliche ADAME während einer Zeitspanne von 2 bis 8 Stunden zugegeben wird.

7. Verfahren nach einem der Ansprüche 1 bis 6, **dadurch gekennzeichnet, dass** die Umsetzung mit einem Molverhältnis von Quaternisierungsmittel und ADAME von 1 bis 1,1 und vorzugsweise 1 bis 1,05 durchgeführt wird.

8. Verfahren nach einem der Ansprüche 1 bis 7, **dadurch gekennzeichnet, dass** die Umsetzung mit einem mittleren Durchsatzverhältnis Wasser/Quaternisierungsmittel von 0,2 bis 1,5; einem mittleren Durchsatzverhältnis restliches ADAME/Quaternisierungsmittel von 2,5 bis 5; und einem mittleren Durchsatzverhältnis Wasser/ADAME von 0,2 bis 1,2 durchgeführt wird.

9. Verfahren nach einem der Ansprüche 1 bis 8, **dadurch gekennzeichnet, dass** es zu einer wässrigen Lösung mit einer Konzentration des quartären Salzes (I) von 50 bis 85 Gew.-% führt.

10. Verfahren nach einem der Ansprüche 1 bis 9, **dadurch gekennzeichnet, dass** es in Gegenwart mindestens eines Stabilisierungsmittels durchgeführt wird, das unter 3,5-Di-*t*-Butyl-4-hydroxytoluol, Hydrochinonmethylether, Hydrochinon, Catechin, *t*-Butylcatechin, Phenothiazin und den Gemischen dieser Stabilisierungsmittel ausgewählt ist, wobei der Mengenanteil des Stabilisierungsmittels oder der Stabilisierungsmittel insbesondere im Bereich von 20 bis 2000 ppm und vorzugsweise 100 bis 1200 ppm, bezogen auf die wässrige Lösung des quartären Salzes (I), liegt.

11. Verfahren nach Anspruch 10, **dadurch gekennzeichnet, dass** es ferner in Gegenwart mindestens eines Maskierungsmittel für Metalle durchgeführt wird, das insbesondere unter Diethylentriaminpentaessigsäure, dem Pentanatriumsalz von Diethylentriaminpentaessigsäure, N-Hydroxyethyl-ethylendiamintriessigsäure und dem Trinatriumsalz von N-Hydroxyethyl-ethylendiamintriessigsäure durchgeführt wird, wobei der Mengenanteil des Maskierungsmittels oder der Maskierungsmittel insbesondere im Bereich von 1 bis 100 ppm und vorzugsweise 5 bis 30 ppm, bezogen auf die wässrige Lösung des quartären Salzes (I), liegt.

12. Verfahren nach einem der Ansprüche 1 bis 11, **dadurch gekennzeichnet, dass** das restliche Quaternisierungsmittel durch Luft-Strippen entfernt wird.

## Claims

1. Process for the manufacture of aqueous solutions of unsaturated quaternary ammonium salts corresponding to the following formula (I): 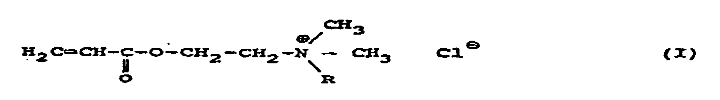 in which R represents a methyl or benzyl radical,
by reaction, in the presence of water, of N,N-dimethyl-aminoethyl acrylate (DAMEA) with a quaternizing agent of formula (II):
R - Cl (II)
in which R is as defined above,
**characterized in that**:
(a) the reaction is carried out in a closed reactor, which comprises 5-60% of the amount by weight of the DAMEA necessary for the reaction and which has been pressurized by air or depleted air to 0.5 to 3 bar, by continuously introducing, at a temperature of 35 to 65°C, on the one hand, the quaternizing agent (II) and, on the other hand, the water and finally the remaining DAMEA, until the desired concentration of salt (I) in the water is obtained,
• the start of the introduction of the water beginning when 0-30% of the amount by weight of the quaternizing agent (II) necessary for the reaction has been added;
• the start of the introduction of the remaining DAMEA beginning when 20-80% of the amount by weight of the quaternizing agent (II) necessary for the reaction has been added; and
• it being possible for the pressure at the end of the reaction to reach 9 bar; then
(b) the reactor is depressurized while keeping the oxygen content constant by simultaneous introduction of air and, after returning to atmospheric pressure, the residual quaternizing agent is removed.

2. Process according to Claim 1, **characterized in that** the reaction is carried out at a temperature of 40 to 60°C.

3. Process according to either of Claims 1 and 2, **characterized in that** the reaction is carried out with a pressure which, at the end of the reaction, reaches 4 to 7 bar.

4. Process according to one of Claims 1 to 3, **characterized in that** the introduction of water is started when 10-20% of the amount by weight of the quaternizing agent (II) necessary for the reaction has been added.

5. Process according to one of Claims 1 to 4, **characterized in that** the introduction of the remaining DAMEA is started when 30-70% of the amount by weight of the quaternizing agent (II) necessary for the reaction has been added.

6. Process according to one of Claims 1 to 5, **characterized in that** the quaternizing agent is introduced over a period of time of 1-7 hours, the water over a period of time of 1-8 hours and the remaining DAMEA over a period of time of 2-8 hours.

7. Process according to one of Claims 1 to 6, **characterized in that** the reaction is carried out with a molar ratio of the quaternizing agent to the DAMEA of 1 to 1.1, preferably of 1 to 1.05.

8. Process according to one of Claims 1 to 7, **characterized in that** the reaction is carried out with a mean ratio of water/quaternizing agent throughput of 0.2-1.5; a mean ratio of remaining DAMEA/quaternizing agent throughput of 2.5-5; and a mean ratio of water/remaining DAMEA throughput of 0.2-1.2.

9. Process according to one of Claims 1 to 8, **characterized in that** it results in an aqueous solution having a concentration of quaternary salt (I) of 50 to 85% by weight.

10. Process according to one of Claims 1 to 9, **characterized in that** it is carried out in the presence of at least one stabilizer chosen in particular from 3,5-di(tert-butyl)-4-hydroxytoluene, hydroquinone methyl ether, hydroquinone, catechol, tert-butylcatechol, phenothiazine and mixtures of these stabilizers, the content of stabilizing agent(s) being in particular from 20 to 2 000 ppm, preferably from 100 to 1 200 ppm, with respect to the aqueous solution of quaternary salt (I).

11. Process according to Claim 10, **characterized in that** it is carried out in the presence in addition of at least one sequestering agent for metals chosen in particular from diethylenetriaminepentaacetic acid, the pentasodium salt of diethylenetriaminepentaacetic acid, N-(hydroxyethyl)ethylenediaminetriacetic acid and the trisodium salt of N-(hydroxyethyl)ethylenediaminetriacetic acid, the content of sequestering agent(s) being in particular from 1 to 100 ppm, preferably from 5 to 30 ppm, with respect to the aqueous solution of quaternary salt (I).

12. Process according to one of Claims 1 to 11, **characterized in that** the residual quaternizing agent is removed by stripping with air.
